# EUROPEAN PATENT SPECIFICATION

(11) **EP 1 919 356 B1**
(45) Date of publication and mention of the grant of the patent: **13.06.2012**
(21) Application number: 06795879.3
(22) Date of filing: 01.09.2006
(51) Int. Cl.: A61B 5/053, A61B 5/103

(54) **METHODS FOR MEASURING MOISTURE CONTENT OF SKIN**
VERFAHREN ZUR MESSUNG DES FEUCHTIGKEITSGEHALTS DER HAUT
PROCEDES DE MESURE DU TAUX D'HYDRATATION DE LA PEAU

(30) Priority: 02.09.2005 US 713813 P
(43) Date of publication of application: 14.05.2008
(73) Proprietor: The Procter & Gamble Company, Cincinnati, OH 45202 (US)
(72) Inventor: SHERMAN, Faiz, Feisal, Mason, Ohio 45040 (US); GARTSTEIN, Vladimir, Mason, Ohio 45040 (US); MOORE, David, Burton, Hamilton, Ohio 45013 (US); MARGRAF, Carl, Hinz, III, 60325 Frankfurt am Main (DE); FISHER, Brian, Keith, Cincinnati, Ohio 45249 (US)
(74) Representative: Marollé, Patrick Pierre Pascal
(86) International application number: PCT/IB2006/053076
(87) International publication number: WO 2007/026335

(56) References cited:
- EP-A1- 0 315 854
- WO-A-2004/042510
- US-A- 5 938 593

## Description

### FIELD OF THE INVENTION

The present invention relates generally to methods for measuring moisture content of skin. The present invention also relates to methods for measuring moisture content of scalp and methods of treating the scalp. Another aspect of the present invention, relates to methods for measuring dandruff conditions on the scalp of a consumer.

### BACKGROUND OF THE INVENTION

Skin is subject to insults by many extrinsic and intrinsic factors. Extrinsic factors include ultraviolet radiation (e.g., from sun exposure), environmental pollution, wind, heat or infrared radiation (IR), low humidity, harsh surfactants, abrasives, and the like. Intrinsic factors include chronological aging and other biochemical changes from within the skin. Whether extrinsic or intrinsic, these factors result in visible signs of skin aging and environmental damage, such as wrinkling and other forms of roughness (including increased pore size, flaking and skin lines), and other histological changes associated with skin aging or damage. Additionally, the water content of the stratum corneum has a profound influence on the appearance, flexibility, texture, and dryness of the skin, and also on the absorption of drugs and other molecules into and through the skin. The stratum corneum is the outermost layer of the epidermis, and comprises the surface of the skin.

Methods of treating the skin generally involve the application of at least one of a variety of appropriate treatments. Such treatments may be selected to provide or to restore certain desired physical or cosmetic characteristics to the skin or scalp. However, unless an appropriate treatment is selected, the desired physical or cosmetic characteristic may not be obtained.

In the case of treating skin, such as the scalp, treatments generally include shampoos, conditioners, colorants, styling compositions, and the like. Manufacturers of these scalp treatments may provide multiple versions of a type or brand of scalp treatment, wherein each of the multiple versions is specifically designed to target a need or demand which is characteristic of a specific consumer segment and which may be based on physical or cosmetic differences of the scalp generally found between respective consumer segments. For example, a single brand of shampoo may offer a first version designed to treat flakes and a second version designed to treat dryness, both conditions associated with dandruff.

However, when a consumer is faced with the task of selecting a scalp treatment from among the multiple versions of a scalp care brand, the consumer may unknowingly select a version which is not designed to provide the characteristics desired by the consumer. In such a case, the consumer may be dissatisfied with the results of the selected version of the scalp care brand. As a result of the consumer's dissatisfaction, the consumer subsequently may refuse to select any of the versions of that same scalp care brand even though another version of that scalp care brand may provide the consumer's desired scalp and/or hair characteristics. The occurrence of such circumstances, in turn, may lead to unnecessary loss of sales of the particular scalp care brand for the manufacturer.

Methods of measuring moisture content have been developed in the past to determine the moisture level of skin or hair, and have relied on various techniques including resistance and capacitance measurements to obtain the desired indication.

The US Patent 5 938 593 is describing a skin condition analyzer with selectable modes of operation includes a probe apparatus for generating a skin representative of the moisture content of the skin and a processor that adjusts the skin condition signal in accordance with environmental components, such as temperature and humidity, to arrive at an overall skin condition signal. The modes of operation include single point random measuring and multiple points patterned measuring for compiling a composite skin analysis report.

### SUMMARY OF THE INVENTION

It has now been discovered that an accurate and repeatable assessment of hydration characteristics of the skin, including the scalp, is achieved by comparing the value of a parameter, such as skin moisture content, at a first position on the skin, such as a control area that is in equilibrium with environment, with the value of the parameter at a second position, such as a test area. By comparing the at least two measured values with each other, as opposed to comparing a single measured value with a known control value, a measured parameter differential profile is obtained which is correlated to a hydration or moisture characteristic assessment. This assessment is independent of the effects of such variables as environmental conditions and subject variability during measurement, because the correlation is based upon the relative differences between the at least two values for each corresponding area on the skin.

In one embodiment, the present invention is directed to a method for measuring moisture content of skin. The method comprises the steps of: (a) providing a means for measuring skin moisture content; (b) using the means for measuring skin moisture content to obtain at least two measured moisture content values for the skin characterized in that the method further comprises the step of; (c) comparing the at least two measured moisture content values with each other to obtain a measured moisture content differential; and (d) correlating the measured moisture content differential to a skin moisture value for the skin.

These and other features, aspects and advantages of the present invention will become evident to those skilled in the art from a reading of the present disclosure.

### BRIEF DESCRIPTION OF THE DRAWINGS

While the specification concludes with claims which particularly point out and distinctly claim the invention, it is believed the present invention will be better understood from the following description taken in conjunction with the accompanying drawings.
FIG. 1 is a flow diagram of the process of an embodiment of the present invention; and
FIG. 2 is a flow diagram of the process for measuring moisture content of the scalp of an embodiment of the present invention.

### DETAILED DESCRIPTION OF THE INVENTION

As used herein, the term "skin" refers to the membranous tissue forming the external covering of a mammalian body including, for example, the external covering of the face, neck, chest, back, arms, hands, legs and scalp.

As used herein, "moisture content" refers to the water present in the skin.

As used herein, "control area" refers to an area of the skin that is exposed to the environment and convenient for measurement.

As used herein, "test area" refers to the area of skin that a consumer wishes to have tested in order to select an appropriate skin treatment for use by consumer on that area.

According to the present invention, methods are described for measuring the moisture content of skin and methods of treating skin. The determination of moisture content in skin, including scalp, is used to quantify various physical and cosmetic characteristics of the skin. For example, a scalp with a low moisture content is unhealthy and may exhibit signs of dandruff, including flakes, dryness, tightness, itchiness and/or redness/irritation. Skin hydration is a function of its normal biological activity that results in continuous moisture flux from within the body to the environment. The improvement of skin barrier function results in greater skin hydration and less moisture loss. As a result, the physical and cosmetic characteristics of the skin may be improved, for example, with treatments that restore skin to normal conditions and improve its barrier function. Improvement of barrier function, in turn, also results in protecting the skin from environmental, physical, chemical, or biological insults and results in an overall improvement in skin health.

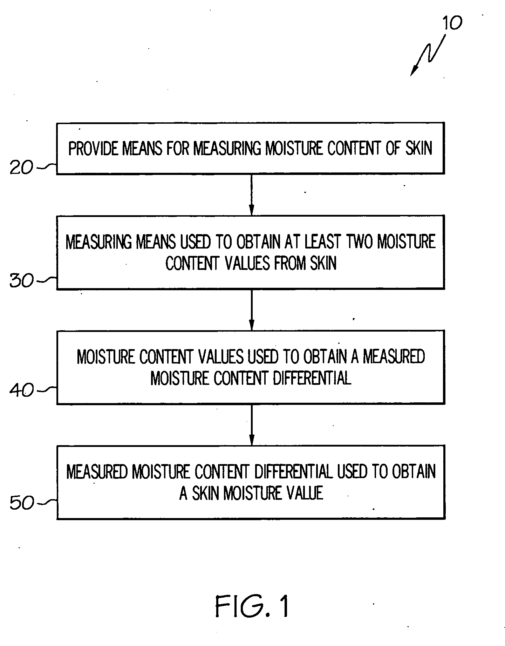

Referring now to FIG. 1, method for measuring moisture content of skin 10, contains a series of steps which are intended to provide information to a recipient or user for purposes of assessing the moisture level of skin. In a first step 20, a means for measuring skin moisture content is provided. Any suitable means for measuring skin moisture content may be used. Suitable means for measuring moisture of skin also include radio frequency, infrared, nuclear magnetic resonance, mechanical vibration, skin deformation, iontophoresis, topology, friction, trans epidermal water loss (TEWL), optical and heat dissipation.

In a second step 30, the means for measuring skin moisture content is used to obtain at least two measured moisture content values from skin. In one embodiment, measurement of the moisture content of the skin is made for at least two areas on the skin, a control area that is in equilibrium with the environment and a test area. In one example, the control area may include the face, neck, ears, arms, hands, legs, feet, abdomen, back and groin, while the test area may include, for example, the face, neck, chest, back, arms, hands, feet, legs and scalp. However, several such measurements at various positions on the test area can be produced. In one example, the means for measuring skin moisture content is used to obtain one or more additional measured moisture content values on the test area. As each measurement is captured by the measurement means it is displayed or stored in a suitable manner. In one example, the measured moisture content values are displayed on an LED display. In another example, the measured moisture content values are stored in a programmable integrated circuit. In one embodiment, the measured values obtained for the control area and the test area have a frequency of less than about 5 MHz, in another embodiment, less than about 2.4 MHz, and in yet another embodiment, less than about 0.5 MHz.

In a third step 40, the at least two measured moisture content values are compared to each other in order to obtain a measured moisture content differential. For example, the skin moisture content value for the control area (m1) is compared to the skin moisture content value for the test area (m2), to obtain a measured moisture content differential (m2 - m1) or (m1 - m2). In one embodiment, the measurement means is electrically coupled through a cable to a processing system, such as a conventional PC or laptop computer. In another embodiment, the measurement means is electrically coupled to a programmable integrated circuit. In yet another embodiment, the measurement means is electrically coupled to a computer wirelessly. The processing system is operable to convert the measured moisture content values generated by the measurement means into a measured moisture content differential. In another embodiment, the measured moisture content differential may be determined manually using the formula (m2 - m1) or (m1-m2).

If additional test area skin moisture values are obtained, all of these values are compared to one another and the measurement with the largest value, lowest value or mean value is then compared with the skin moisture content value for the control area in order to obtain a measured moisture content differential profile. In one embodiment, a measurement differential from about 0 MHz to about 0.05 MHz, represents a healthy skin condition (high moisture level); a measurement differential from about 0.05 MHz to about 0.5 MHz represents a moderate skin condition (medium moisture level); and a measurement differential greater than about 0.5 MHz represents an unhealthy skin condition (low moisture level).

In another embodiment, the control area and the test area could be the same. For example, a measurement of the moisture content is taken at the control area. A period of time is then allowed to elapse during which the control area may be treated with a skin or scalp treatment. Thereafter, a second measurement is taken at the control area. The two measured moisture content values are then compared to each other in order to obtain a measured moisture content differential. In effect, the control area and the test area are the same for the second measurement.

In a fourth step 50, the moisture content differential is correlated to a skin moisture value. In one embodiment, the processing system described above is operable to convert the frequency differential into a skin moisture value as shown in Table I.

**TABLE I**

| **Measured Signal Difference between Control Area and Test Area (MHz)** | Skin Moisture Value | Skin Condition |
|---|---|---|
| >=0.9 | 5 | D |
| 0.79 - 0.89 | 10 | D |
| 0.68 - 0.78 | 20 | D |
| 0.54 - 0.67 | 30 | D |
| 0.43 - 0.53 | 40 | D |
| 0.34 - 0.42 | 50 | M |
| 0.25 - 0.33 | 60 | M |
| 0.15 - 0.24 | 70 | M |
| 0.06 - 0.14 | 80 | M |
| 0.04 - 0.05 | 90 | H |
| <= 0.03 | 95 | H |

In one embodiment, the skin moisture value is from about 0 to about 99, and in another embodiment, from about 5 to about 95. Such a value is based upon the relative differences between the measured values for the control area and the test area of the skin. As such, the skin serves as its own control. The skin moisture value may also be represented by such terms as "healthy" or "H", "moderate" or "M", and "dry" or "D," however, any words, pictures, or numbered grade scales which depict increasing or decreasing quantities of skin moisture levels may be used in the invention.

In another aspect, the invention relates, additionally, to using the skin moisture value to select at least one appropriate skin treatment. As used herein, an "appropriate skin treatment" is a chemical composition or non-chemical treatment which provides or restores skin with the physical or cosmetic characteristics desired by a consumer. Exemplary skin treatment chemical compositions may include, without limitation, beauty care products, healthcare products, cosmetics, baby care products, feminine care products and pet care products, in the form of, lotions, creams, gels, tonics, after shave, sticks, sprays, ointments, pastes, powders, mousse, shampoos, conditioners, oils, colorants, and biomedical and dermatological treatments.

Nonlimiting examples of biomedical and dermatological treatments include prescription skin care treatments, laser treatment, chemical peel, dermabrasion, electrical stimulation, botox treatments, and exfoliating pads and cloths.

Exemplary non-chemical treatment may include, without limitation, hair shaving and hair removal.

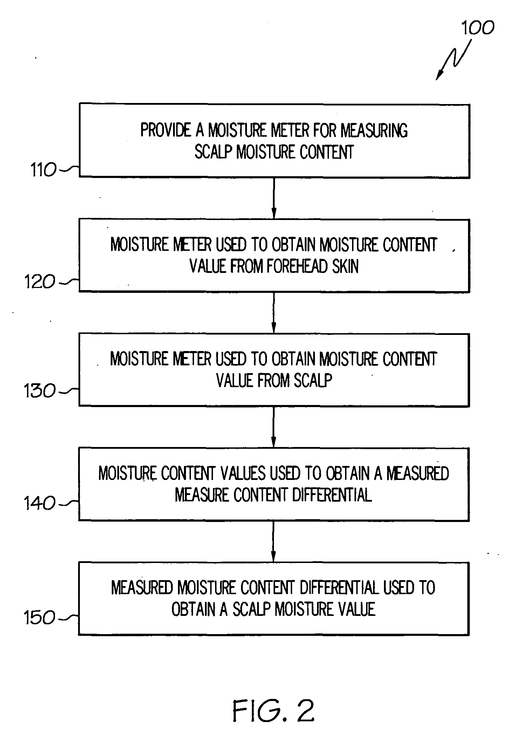

Referring now to FIG. 2, method for measuring moisture content of the scalp 100, contains a series of steps which are intended to provide information to a recipient or user for purposes of assessing the moisture level of the scalp. In a first step 110, a moisture meter for measuring scalp moisture content is provided. In one embodiment, the moisture meter may be an electronic device comprising an impedance sensor, as described above. In a second step 120, the moisture meter is used to obtain at least a first measured moisture content value at a position off the scalp. In one example, the off the scalp location may include, for example, forehead skin, cheek, chin, ear and arm.

In a third step 130, the moisture meter is used to obtain at least a second measured moisture content value at a position on the scalp. With respect to at least a second measured moisture value, several such measurements at various positions on the scalp can be produced. In one example, the moisture meter for measuring scalp moisture content is used to obtain one or more additional measured moisture content values at various locations on the scalp. As each measured moisture content value is captured in steps 120 and 130, the values are displayed in a suitable manner, for example, on an LED display on the moisture meter. In one embodiment, the moisture content values obtained for the position off the scalp location and the position on the scalp have a frequency of less than about 5 MHz, in another embodiment less than about 2.4 MHz, and in yet another embodiment less than about 0.5 MHz.

In a fourth step 140, the first measured moisture content value is compared with the second measured moisture content value to obtain a measured moisture content differential. For example, the scalp moisture content value for the position off the scalp (m1) is compared to the scalp moisture content value for the position on the scalp (m2), to obtain a measured moisture content differential (m2 - m1) or (m1 - m2). The moisture meter may be electrically coupled as described above. The processing system is operable to convert the measured moisture content values generated by the meter into a measured moisture content differential. In another embodiment, the measured moisture content differential may be determined manually using the formula (m2 - m1) or (m1 -m2).

If additional scalp moisture values are obtained at positions on the scalp; all of these values are compared to one another. The scalp moisture value with the largest value, or lowest value or mean value is then compared with the moisture content value for the position off the scalp. In one embodiment, a measurement differential from about 0 MHz to about 0.05 MHz, represents a healthy scalp condition (high moisture level); a measurement differential from about 0.05 MHz to about 0.5 MHz represents a moderate scalp condition (medium moisture level); and a measurement differential greater than about 0.5 MHz represents an unhealthy scalp condition (low moisture level).

In a fifth step 150, the moisture content differential is correlated to a scalp moisture value, in the same manner as described above with respect to the skin moisture value.

In another aspect of the invention, the method of this invention relates to a method of treating the scalp, including (a) measuring moisture content of said scalp according to the method described above; (b) using the correlated scalp moisture value to select at least one appropriate scalp treatment; and (c) applying the appropriate scalp treatment to the scalp. As used herein, an "appropriate scalp treatment" is a treatment which provides or restores the scalp with the physical or cosmetic characteristics desired by a consumer. Exemplary scalp treatment compositions may include, without limitation, shampoos, conditioners, oils, colorants, and styling compositions.

### EXAMPLE

### Example 1. Scalp Moisture Content and Dandruff

To determine a correlation between moisture content of scalp and dandruff, the scalp moisture content of consumers was measured using an electronic device comprising an impedance sensor as described above. The scalp moisture content for each consumer was determined as follows: One reading was taken from the forehead; and two readings were taken from the scalp. The measured moisture content values were then converted into a measured moisture content differential by the device. In addition, each consumer was assigned an Adherent Scalp Flaking Score ("ASFS"). The ASFS for each consumer was determined by having a qualified grader examine an octant of the consumer's scalp and then assigning a flake grade to that octant. Exemplary results are shown below Table II.

**TABLE II**

| Number of Consumers | Measured Signal Difference between m1 and m2 (KHz) | ASFS Octant Grade | Scalp Condition |
|---|---|---|---|
| 61 | 395.164 | 8-10 | Severe Flaking |
| 235 | 308.766 | 4-6 | Moderate Flaking |
| 169 | 230.527 | 0-2 | No Flaking |

## Claims

1. A method for measuring moisture content of skin, said method comprising the steps of:
a) providing a means for measuring skin moisture content (20);
b) using said means for measuring skin moisture content to obtain at least two measured moisture content values for said skin (30) **characterized in that** the method further comprises the steps of;
c) comparing said at least two measured moisture content values with each other to obtain a measured moisture content differential (40); and
d) correlating said measured moisture content differential to a skin moisture value for said skin (50).

2. The method according to claim 1, wherein said means for measuring skin moisture content is an electronic device.

3. The method according to claim 2, wherein said electronic device comprises an impedance sensor.

4. The method according to claim 1, wherein step (b) comprises obtaining a first measured moisture content value from a control area that is in equilibrium with environment and a second measured moisture content value on a test area.

5. The method according to claim 4, wherein said control area is selected from the group consisting of face, neck, ears, arms, hands, legs, feet, abdomen, back and groin.

6. The method according to claim 4, wherein said test area is selected from the group consisting of face, neck, chest, back, arms, hands, feet, legs and scalp.

7. The method according to claim 1, further comprising the step of using said skin moisture value to select at least one appropriate skin treatment.

8. The method according to claim 7, wherein said appropriate skin treatment is selected from the group consisting of chemical compositions and non-chemical treatments.

9. The method according to claim 8, wherein said chemical compositions are selected from the group consisting of lotions, creams, gels, tonics, after shave, sticks, sprays, ointments, pastes, powders, mousse, shampoos, conditioners, oils, colorants, and biomedical and dermatological treatments.

10. The method according to claim 8, wherein said non-chemical treatment is selected from the group consisting of hair shaving and hair removal.

11. The method according to claim 4, further comprising obtaining a third measured moisture content value for said skin at a second position on said test area.

12. A cosmetic, non surgical method of treating the scalp, said method comprising the steps of:
a) measuring moisture content of said scalp according to the method of claim 1;
b) using said correlated scalp moisture value to select at least one appropriate scalp treatment; and
c) applying said appropriate scalp treatment to said scalp.

13. The method according to claim 12, wherein said appropriate scalp treatment is selected from the group consisting of shampoos, conditioners, colorants, oils, and styling compositions.

## Patentansprüche

1. Verfahren zum Messen des Feuchtigkeitsgehalts von Haut, wobei das Verfahren die folgenden Schritte umfasst:
a) Bereitstellen eines Mittels zum Messen des Hautfeuchtigkeitsgehalts (20),
b) Verwenden des Mittels zum Messen des Hautfeuchtigkeitsgehalts, um mindestens zwei gemessene Feuchtigkeitsgehaltswerte für die Haut (30) zu erhalten, **dadurch gekennzeichnet, dass** das Verfahren ferner die folgenden Schritte umfasst,
c) Vergleichen der mindestens zwei gemessenen Feuchtigkeitsgehaltswerte miteinander, um ein gemessenes Feuchtigkeitsgehaltsdifferential (40) zu erhalten, und
d) Korrelieren des gemessenen Feuchtigkeitsgehaltsdifferentials mit einem Hautfeuchtigkeitswert für die Haut (50).

2. Verfahren nach Anspruch 1, wobei das Mittel zum Messen des Hautfeuchtigkeitsgehalts eine elektronische Vorrichtung ist.

3. Verfahren nach Anspruch 2, wobei die elektronische Vorrichtung einen Impedanzsensor umfasst.

4. Verfahren nach Anspruch 1, wobei Schritt (b) das Erhalten eines ersten gemessenen Feuchtigkeitsgehaltswerts von einem Kontrollbereich, der in Gleichgewicht mit der Umgebung ist, und eines zweiten gemessenen Feuchtigkeitsgehaltswerts auf einem Prüfbereich umfasst.

5. Verfahren nach Anspruch 4, wobei der Kontrollbereich ausgewählt ist aus der Gruppe bestehend aus Gesicht, Hals, Ohren, Armen, Händen, Beinen, Füßen, Bauch, Rücken und Leiste.

6. Verfahren nach Anspruch 4, wobei der Prüfbereich ausgewählt ist aus der Gruppe bestehend aus Gesicht, Hals, Brust, Rücken, Armen, Händen, Füßen, Beinen und Kopfhaut.

7. Verfahren nach Anspruch 1, ferner umfassend den Schritt des Verwendens des Hautfeuchtigkeitswerts zum Auswählen mindestens einer angemessenen Hautbehandlung.

8. Verfahren nach Anspruch 7, wobei die angemessene Hautbehandlung ausgewählt ist aus der Gruppe bestehend aus chemischen Zusammensetzungen und nicht chemischen Zusammensetzungen.

9. Verfahren nach Anspruch 8, wobei die chemischen Zusammensetzungen ausgewählt sind aus der Gruppe bestehend aus Lotionen, Cremes, Gelen, Tonika, Aftershave, Sticks, Sprays, Salben, Pasten, Pulvern, Schaum, Shampoos, Spülungen, Ölen, Farbmitteln und biomedizinischen und dermatologischen Behandlungen.

10. Verfahren nach Anspruch 8, wobei die nicht chemische Behandlung ausgewählt ist aus der Gruppe bestehend aus Haarrasur und Haarentfernung.

11. Verfahren nach Anspruch 4, umfassend das Erhalten eines dritten gemessenen Feuchtigkeitsgehaltswerts für die Haut an einer zweiten Position auf dem Prüfbereich.

12. Kosmetisches, nicht chirurgisches Verfahren zum Behandeln der Kopfhaut, wobei das Verfahren die folgenden Schritte umfasst:
a) Messen des Feuchtigkeitsgehalts der Kopfhaut gemäß dem Verfahren nach Anspruch 1,
b) Verwenden des korrelierten Kopfhautfeuchtigkeitswerts, um mindestens eine angemessene Kopfhautbehandlung auszuwählen, und
c) Auftragen der angemessenen Kopfhautbehandlung auf die Kopfhaut.

13. Verfahren nach Anspruch 12, wobei die angemessene Kopfhautbehandlung ausgewählt ist aus der Gruppe bestehend aus Shampoos, Spülungen, Farbmitteln, Ölen und Frisierzusammensetzungen.

## Revendications

1. Procédé pour mesurer la teneur en humidité de la peau, ledit procédé comprenant les étapes consistant à :
a) fournir un moyen pour mesurer la teneur en humidité de la peau (20) ;
b) utiliser ledit moyen pour mesurer la teneur en humidité de la peau de façon à obtenir au moins deux valeurs de teneur en humidité mesurées pour ladite peau (30) **caractérisé en ce que** le procédé comprend en outre les étapes consistant à ;
c) comparer lesdites au moins deux valeurs de teneur en humidité mesurées l'une par rapport à l'autre de façon à obtenir une différence de teneur en humidité mesurée (40) ; et
d) corréler ladite différence de teneur en humidité mesurée à une valeur d'humidité de peau pour ladite peau (50).

2. Procédé selon la revendication 1, dans lequel ledit moyen pour mesurer la teneur en humidité de la peau est un dispositif électronique.

3. Procédé selon la revendication 2, dans lequel ledit dispositif électronique comprend un capteur d'impédance.

4. Procédé selon la revendication 1, dans lequel l'étape (b) comprend l'obtention d'une première valeur de teneur en humidité mesurée provenant d'une zone témoin qui est en équilibre avec l'environnement et une deuxième valeur de teneur en humidité mesurée sur une zone de test.

5. Procédé selon la revendication 4, dans lequel ladite zone témoin est choisie dans le groupe constitué du visage, du cou, des oreilles, des bras, des mains, des jambes, des pieds, de l'abdomen, du dos et de l'aine.

6. Procédé selon la revendication 4, dans lequel ladite zone de test est choisie dans le groupe constitué du visage, du cou, de la poitrine, du dos, des bras, des mains, des pieds, des jambes et du cuir chevelu.

7. Procédé selon la revendication 1, comprenant en outre l'étape consistant à utiliser ladite valeur d'humidité de peau pour sélectionner au moins un traitement approprié de la peau.

8. Procédé selon la revendication 7, dans lequel ledit traitement approprié de la peau est choisi dans le groupe constitué de compositions chimiques et traitements non chimiques.

9. Procédé selon la revendication 8, dans lequel lesdites compositions chimiques sont choisies dans le groupe constitué de lotions, crèmes, gels, toniques, après-rasage, bâtonnets, aérosols, onguents, pâtes, poudres, mousses, shampooings, conditionneurs, huiles, colorants, et traitements biomédicaux et dermatologiques.

10. Procédé selon la revendication 8, dans lequel ledit traitement non chimique est choisi dans le groupe constitué de rasage des poils et épilation.

11. Procédé selon la revendication 4, comprenant en outre l'obtention d'une troisième valeur de teneur en humidité mesurée pour ladite peau à une deuxième position sur ladite zone de test.

12. Procédé cosmétique non chirurgical de traitement du cuir chevelu, ledit procédé comprenant les étapes consistant à :
a) mesurer la teneur en humidité dudit cuir chevelu selon le procédé selon la revendication 1 ;
b) utiliser ladite valeur d'humidité du cuir chevelu corrélée pour sélectionner au moins un traitement approprié du cuir chevelu ; et
c) appliquer ledit traitement approprié du cuir chevelu audit cuir chevelu.

13. Procédé selon la revendication 12, dans lequel ledit traitement approprié du cuir chevelu est choisi dans le groupe constitué de shampooings, conditionneurs, colorants, huiles, et compositions de coiffage.
